# EUROPEAN PATENT APPLICATION

(11) **EP 1 842 850 A1**
(43) Date of publication of application: **10.10.2007**
(21) Application number: 06005969.8
(22) Date of filing: 23.03.2006
(51) Int. Cl.: C07D 417/12, A61K 31/427, A61P 3/10

(54) **Rosiglitazone hydrochloride hemihydrate**

(71) Applicant: SANDOZ AG, 4056 Basel (CH)
(72) Inventor: Wieser, Josef, 6403 Polling (AT); Pichler, Arthur, 6200 Jenbach (AT); Lengauer, Hannes, 6250 Kundl (AT); Kingler, Elfriede, 6311 Wildschönau (AT)
(74) Representative: Dietz, Jörg-Reimar

(57) **Abstract**

5-[4-[2-(N-methyl-N-(2-pyridyl)amino)ethoxy]benzyl]thiazolidine-2,4-dione, hydrochloride hemihydrate (rosiglitazone hydrochloride hemihydrate) characterized in that: (i) comprises water in the range of from about 1.0 to about 2.5% w/w; and (ii) provides an infrared spectrum containing peaks at about 3504, 2751, 1688, 1601 and 1215 cm-1; and/or (iii) provides an X-ray powder diffraction (XRPD) pattern containing peaks at about 12.5, 17.1, 21.3, 26.7, 27.7 °2 θ; a process for preparing such a compound, a pharmaceutical composition containing such a compound and the use of such a compound in the treatment and/or prophylaxis of diabetes mellitus.

## Description

### Field of the invention

This invention relates to a novel compound, to a process for the preparation of said compound, to pharmaceutical compositions containing said compound and to the use of such a compound and of such compositions in medicine.

### Background of the invention

European Patent Application, Publication Number 0 306 228 relates to certain thiazolidinedione derivatives as having hypoglycaemic and hypolipidaemic activity. The compound of example 30 of EP 0 306 228 A1 is 5-[4-[2-(N-methyl-N-(2-pyridyl)amino)ethoxy]benzyl]thiazolidine-2,4-dione (according to Merck Index, 13^{th} Edition, Monograph number 8346, CAS Registry number: 122320-73-4), i.e. rosiglitazone as generic name.

International Patent Application, Publication Number WO 94/05659 discloses certain salts of the compounds of EP 0 306 228. The preferred salt of WO 94/05659 is the maleic salt of rosiglitazone.

International Patent Applications, Publication Number WO 01/68646 and WO 02/20519 each disclose distinct anhydrous forms of rosiglitazone hydrochloride and International Patent Application, Publication Number WO 00/63205 discloses a dihydrate and International Patent Application, Publication Number WO 00/63206 discloses a monohydrate of rosiglitazone hydrochloride.

### Description of the invention

Because of easy availability and for physiological tolerability rosiglitazone hydrochloride salt with suitable properties for pharmaceutical processing on a commercial scale remains an alternative salt form. It has been discovered that rosiglitazone hydrochloride exists in the form of a novel rosiglitazone hydrochloride salt which is hemihydrated. This novel rosiglitazone hydrochloride hemihydrate (hereinafter also referred to as "Hydrochloride Hemihydrate") is particularly suitable for bulk preparation and handling. The novel form is stable, non-hygroscopic, possesses good bulk flow properties and can be prepared by an efficient, economic and reproducible process particluarly suited to large-scale preparation.

The novel Hydrochloride Hemihydrate also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione, hydrochloride hemihydrate characterized in that it:
(i) comprises water in the range of from about 1.0 to about 2.5% w/w; and
(ii) provides an infrared spectrum containing peaks at about 3504, 2751, 1688, 1601 and 1215 cm⁻¹; and/or
(iii) provides an X-ray powder diffraction (XRPD) pattern containing peaks at about 12.5, 17.1, 21.3, 26.7, 27.7 °2 θ.

In one favoured aspect, Hydrochloride Hemihydrate provides an infra red spectrum substantially in accordance with Figure I.

In one favoured aspect, Hydrochloride Hemihydrate provides an X-ray powder diffraction (XRPD) pattern substantially in accordance with Table 2 and Figure II.

The Hydrochloride Hemihydrate may exist in certain dehydrated forms which reversibly convert to the Hydrochloride Hemihydrate when contacted with water, either in liquid or vapour form. The present invention encompasses all such reversibly rehydratable forms of the Hydrochloride Hemihydrate. Preferably, there is provided the hydrated form as characterized above.

The present invention encompasses Hydrochloride : Hemihydrate isolated in pure crystalline form or when admixed with other materials.

Thus in one aspect there is provided Hydrochloride Hemihydrate in isolated form.
In a further aspect there is provided Hydrochloride Hemihydrate in pure form.
In yet a further aspect there is provided Hydrochloride Hemihydrate in crystalline form.

The invention also provides a process for preparing Hydrochloride Hemihydrate (rosiglitazone hydrochloride hemihydrate) which comprises the steps of:
(i) reacting 5-[4<2-(N-methyl-N-(2-pyridyl)arriino)ethoxy]benzyl]thiazolidine-2,4-dione or an acid addition salt thereof in the presence of a suitable organic solvent and a suitable amount of water for formation of rosiglitazone hydrochloide hemihydrate, with the source of hydrogen chloride;
(ii) the obtained solution is cooled to a temperature in the range of from about 0°C to about 30°C to complete crystallisation;
(iii) recovery of rosiglitazone hydrochloride hemihydrate in pure crystalline form.

The starting compound 5-[4-(2-(N-methyl-N-(2-pyridyt)amino)ethoxy]benzyt]thiazo!idine-2,4-dione (rosiglitazone) may be prepared according to the description of EP 0 306 228 A1. A suitable acid addition salt of rosiglitazone may also be used as starting compound, e.g. its maleate salt or hydrochloride salt.

A suitable organic solvent may be C₁-C₄ alkyl alcohol, e.g. ethanol, n-propanol, propan-2-ol (isopropanol), n-butanol, isobutanol, tert-butanol or nitrile such as acetonitrile. The reaction of both starting compounds in a suitable organic solvent and a suitable amount of water for forming rosiglitazone hydrochloride hemihydrate may be carried out at elevated temperature, e.g. in the range of from about 60°C to about 80°C, preferably form about 65°C to about 75°C, more preferably about 70°C. Optionally the crystallisation may be initiated by seeding of the reaction mixture with crystals of the Hydrochloride Hemihydrate. Concentrated hydrochloric acid is preferred source of hydrogen chloride, but any other suitable source of hydrogen chloride may be used, providing the amount of water in the reaction medium is suitable for formation of the Hydrochloride Hemihydrate.

Recovery of the desired compound comprises crystallisation of Hydrochloride Hemihydrate by cooling the obtained solution of said Hydrochloride Hemihydrate to about ambient temperature, e.g. about 25°C, but preferably the reaction temperature may be further lowered to a temperature in the range from about 0°C to about 5°C to complete crystallisation.

In above mentioned process the total amount of water in the reaction mixture is preferably in the range of from about 1 % to about 8% w/v, preferably for example about 2% w/v.

In another aspect of the invention for preparing Hydrochloride Hemihydrate a suspension of anhydrous 5-[4-(2-(N-methyl-N-(2-pyridyl)amino)ethoxy]benzyl]thiazolidine-2,4-dione hydrochloride salt (prepared according to the disclosure of the patent EP 1 315 723) in a suitable organic solvent, preferably C₁-C₄ alkly alcohol, more preferably propanol-2-ol, and a suitable amount of water for formation of said Hydrochloride Hemihydrate is agitated or stirred at temperature providing the desired compound, e.g. at temperature in the range of from about 20°C to 30°C, preferably at about ambient temperature. The total amount of water in the reaction medium is in the range of from about 1% to about 8% w/v, e.g. about 2% w/v. In said process the conversion may be initiated by seeding the reaction mixture with crystals of Hydrochloride Hemihydrate.

As mentioned above the novel Hydrochloride Hemihydrate has useful therapeutic properties. The present invention accordingly provides the Hydrochloride Hemihydrate for use as an active therapeutic substance.

More particularly the present invention provides the Hydrochloride Hemihydrate for use in the treatment and/or prophylaxix of diabetes mellitus, conditions associated with diabetes mellitus and certain complications thereof in a human or non-human mammal.

When used herein, the term "prophylaxis of conditions associated with diabetes mellitus" includes treating conditions such as insulin resistance, impaired glucose tolerance, hyperinsulinaemia and gestational diabetes. Diabetes mellitus preferably means Type II diabetes mellitus. Conditions associated with diabetes mellitus include hyperglycaemia, hyperlipidaemia, obesity, hypertension, cardiovascular disease, certain eating disorders, polycystic ovarian syndrome and steroid induced insulin resistance. Complications of conditions associated with diabetes mellitus encompassed herein include renal disease, especially renal disease associated with the development of Type II diabetes mellitus including diabetic nephropathy, glumerulonephritis, glumerular sclerosis, nephrotic sndrome, hypertensive nephrosclerosis and end stage renal disease.

The Hydrochloride Hemihydrate may be administered per se, or preferably as a pharmaceutical composition also comprising a pharmaceutically acceptable carrier.

As used herein, the term "pharmaceutically acceptable" embraces compounds, compositions and ingredients for both human and veterinary use.

In accordance with conventional pharmaceutical practice the carrier may comprise a diluent, filler, disintegrant, wetting agent, lubricant, colourant, flavourant or other conventional adjuvant or excipient. The term "pharmaceutically acceptable carrier" as used herein is intended to include encapsulating material providing a capsule which surrounds the pharmaceutically active substance per se or together with other pharmaceutically acceptable carriers.

The Hydrochloride Hemihydrate may be administered by any suitable route, but usually by the oral or parenteral routes.

Pharmaceutical compositions may be prepared by admixture and are suitably adapted for oral, parenteral or topical administration, and as such may be in the form of tablets, capsules, oral liquid preparations, powders, granules, lozenges, pastilles, reconstitable powders, injectable and infusable solutions or suspensions, suppositories and transdermal devices.

Suitable methods for formulating the pharmaceutical compositions of the Hydrochloride Hemihydrate are known.

Additionally, the present invention provide a pharmaceutical composition comprising the Hydrochloride Hemihydrate in combination with one or more other antidiabetic agents, e.g. biguanides, sulfonylureas and alpha glucosidase inhibitors, and optionally with a pharmaceutically acceptable carrier.

The present invention further provides a method for the treatment and/or prophylaxis of diabetes mellitus, conditions associated with diabetes mellitus and certain complications thereof in a human or non-human mammal, which comprises administering the Hydrochloride Hemihydrate to a human or non-human mammal in a need thereof. The Hydrochloride Hemihydrate are applied in a pharmaceutically effective, non-toxic amount. Pharmaceutically effective amounts within the meaning of the present invention include doses that provide a desirable physiological and/or pharmacological effect.

In the treatment and/or prophylaxis of diabetes mellitus, conditions associated with diabetes mellitus and certain complications thereof, the Hydrochloride Hemihydrate may be taken in amounts so as to provide 5-[4-[2-(N-methyl-2-pyridyl)amino)ethoxylbenzyl]-thiazolidine-2,4-dione in a suitable doses, e.g. such as disclosed in EP 0 306 228 A1.

In a further aspect, the present invention provides the use of the Hydrochloride Hemihydrate *per se,* or comprised in the herein described pharmaceutical compositions, for the manufacture of a medicament for the treatment and/or prophylaxis of diabetes mellitus, conditions associated with diabetes mellitus and certain complications thereof.

Additionally, the present invention provides the use of the Hydrochloride Hemyhydrate in combination with one or more other antidiabetic agents, e.g. biguanides, sulfonylureas and alpha glucosidase inhibitors, for the manufacture of a medicament for the treatment and/or prophylaxis of diabetes mellitus, conditions associated with diabetes mellitus and certain complications thereof.

The following examples illustrate the invention but do not limit it in any way. All temperatures are given in degree Celsius and are uncorrected.

The infrared spectra are recorded using a BRUKER FTIR-Tensor 27 diamond ATR.

The XRPD is recorded on a AXS-BRUKER X-ray powder diffractometer D-8 using the following acquisition conditions: tube anode: Cu; generator tension. 40 kV, generator current: 40 mA, start angle. 2.0°θ; end angle: 40.0°θ; stepsize: 0.01°θ; time per step: 2 seconds.

### Preparation of 5-[4-[2-(N-methyl-N-(2-pyridyl)amino)ethoxy]benzyl]thiazolidine-2,4-dione hydrochloride hemihydrate ("Hydrochloride Hemihydrate")

### Example 1

A mixture of 4.0 g (11.12 mmol) of 5-[4-[2-(N-methyl-N-(2-pyridyl)amino)ethoxy]benzyl]thiazilidine-2,4-dione and 100 ml propan-2-ol is stirred and heated to 70°C. 1.1. ml concentrated hydrochloric acid is added forming a clear solution. After cooling to 30°C a part of the HCl salt precipitated as a viscous like solid. The mixture is stirred at room temperature over night and during this time the insoluble solid partially converts to a crystalline salt. The crystalline part of the product is collected by filtration, washed with propan-2-ol and dried under vacuum to give 2.75 g of the title compound as a white crystalline solid.
Water content (Karl-Fischer): 1.2% w/w.

### Example 2

A mixture of 4 g (11.2 mmol) of 5-[4-[2-(N-methyl-N-(2-pyridyl)amino)ethoxy]benzylJthiazolidine-2,4-dione and 100 ml propan-2-ol containing 5% (v/v) water is stirred and heated to 70°C. 1.1 ml concentrated hydrochloric acid (1.2 equivalent) is added forming a clear solution after 10min. After cooling to 65°C the solution is seeded with crystals of Hydrochloride Hemihydrate. The obtained mixture is colled to 25°C and stirred at this temperatures for two hours. The precipitated product is collected by filtration and dried at 25°C under low vacuum (200 mbar) to give 3.9 g of the title rosiglitazone hydrochloride hemihydrate salt (Hydrochloride Hemihidrate).
Water content (Karl-Fischer): 2.25 w/w.

### Example 3

A mixture of 20.0 g (0.056 mot) of 5-[4-[2-(N-methyl-N-(2-pyridyl)amino)ethoxy]benzyl]thiazolidinedione-2,4-diohe and 500 ml propan-2-ol containing 2% (v/v) water is stirred and heated to 70°C. 5.6 ml concentrated hydrochloric acid (1.2 equivalent) is added forming a clear solution after 15 min. After colling to 65°C the solution is seeded with crystals of Hydrochloride Hemihydrate. The obtained mixture is cooled to 25°C and stirred at this temperature for two hours. The precipitated product is collected by filtration, washed with propan-2-ol and dried at 25°C under low vacuum (200 mbar) to give 20.5 g of the title Hydrochloride Hemihydrate.
Water content (Karl-Fischer): 2.0% w/w.

### Example 4

A mixture of 2 g (0.056 mol) of 5-[4-[2-(N-methyl-N-(2-pyridyl)amino)ethoxy]benzyl]thiazolidine-2,4-dione and 50 ml ethanol is stirred and heated to 70°C. 0.56 ml concentrated hydrochloric acid (1.2 equivalent) is added forming a clear solution after 5 min. After cooling to 45°C the solution is seeded with crystals of Hydrochloride Hemihydrate. The obtained mixture is cooled to 25°C and stirred at this temperature over the night. The precipitated product is collected by filtration and dried at 25°C under low vacuum (200 mbar) to give 1.5 g of the title Hydrochloride Hemihydrate.
Water content (Kari-Fischer): 2.1 % w/w.

### Example 5

A mixture of 1 g (2.8 mmol) of 5-[4-[2-(N-methyl-N-(2-pyridyl)amino)ethoxy]benzyl]thiazolidine-2,4-dione and 24.5 ml acetonitrile and 0.5 ml water is stirred and heated to 70°C. 0.28 ml concentrated hydrochloric acid (1.2 equivalent) is added to the solution and after cooling to 65°C the solution is seeded with crystals of Hydrochloride Hemihydrate. The obtained mixture is cooled to 25°C and stirred at this temperature for two hours. The precipitated product is collected by filtration and dried at 25°C under low vacuum (200 mbar) to give 0.86 g of the title Hydrochloride Hemihydrate.
Water content (Karl-Fischer): 2.2 w/w.

### Example 6

4 g (0.01 mol) of anhydrous 5-[4-[2-(N-niethyl-N-(2-pyridyl)amino)ethoxy]benzyl]thiazolidine-2,4-dione hydrochloride (prepared according to the disclosure of patent EP 1 315 723) in a mixture of 137 ml of propan-2-ol and 3 ml water are heated to reflux temperature. The turbid mixture is hot filtered to obtain a clear solution. The obtained solution is seeded with crystals of Hydrochloride Hemihydrate and then stirred at 25°C for two hours. The precipitated product is collected by filtration and dried at 25°C under vacuum to give 3.36 g of the title Hydrochloride Hemihydrate.
Water content (Karl-Fischer): 2.2% w/w.

### Example 7

A mixture of 2.65 g (5 mmol) of 5-[4-[2-(N-methyl-N-(2-pyridyl)amino)ethoxy]benzyl]thiazolidine-2,4-dione maleate and 65 ml technical ethanol (96 vol %) is stirred and heated to 70°C. The obtained solution is cooled to 25°C and seeded with crystals of Hydrochloride Hemihydrate. After stirring at 25°C over night the precipitated product is collected by filtration and dried at 25°C under low vacuum (200 mbar) to give 1.24 g of the title Hydrochloride Hemihydrate.
Water content (Karl-Fischer): 2.1 % w/w.

### Example 8

A suspension of 2 g (5 mmol) of anhydrous 5-[4-[2-(N-methyl=N-(2-pyridyl)amino)ethoxyJbenzyl]thiazolidine-2,4-dione hydrochloride (prepared according to the disclosure of patent EP 1 315 723) and 0.1 g Hydrochloride Hemihydrate in a mixture of 19.6 ml propan-2-ol and 0.4 ml water is strirred at 25°C over night. The solid is isolated and dried at 25°C under low vacuum (200 mbar) to give 2.0 g of the title Hydrochloride Hemihydrate.
Water content (Karl-Fischer): 2.1 % w/w.

### Characterizing data for the product of Example 3:

The infrared absorption spectrum of the solid product, prepared according to Example 3, is shown in Figure I, and bands observed are as mentioned in the description above.

X-ray powder diffraction (XRPD) pattern of the solid product, prepared according to Example 3, is shown in Figure II, and characteristic XRPD angles (°2 θ) and relative intensities (in %) are recorded in Table 2.

### Hygroscopicity of the Hydrochloride Hemihydrate

### (rosiglitazone hydrochloride hemihydrate)

A sample of the Hydrochloride Hemihydrate, prepared according to Example 3, is exposed to different relative humidities. Results are given below in Table 1

**Table 1**

| | Initial | 40°C / 75% r.h. | 60°C / 100% r.h. |
|---|---|---|---|
| | | 5 days | 24 hrs |
| % H₂O | 2.16 | 2.19 | 2.20 |

Conclusion: The Hydrochloride Hemihydrate (rosiglitazone hydrochloride hemihydrate) is non-hygroscopic.

Characteristic X-Ray Powder Diffraction (XRPD) pattern angles and relative intensities of Hydrochloride Hemihydrate (Table 2)

| Diffraction | Relative |
|---|---|
| Angle (°2 θ) | Intensity (%) |
| 11.5 | 5.7 |
| 12.5 | 27.4 |
| 13.3 | 21.7 |
| 13.8 | 17.7 |
| 14.0 | 12.5 |
| 17.1 | 61.0 |
| 17.9 | 8.7 |
| 18.8 | 20.3 |
| 19.7 | 9.6 |
| 20.0 | 11.9 |
| 21.3 | 100.0 |
| 21.8 | 26.8 |
| 22.5 | 19,9 |
| 23.2 | 15.9 |
| 23.5 | 20.6 |
| 25.3 | 12.5 |
| 26.4 | 23.4 |
| 26.7 | 51.3 |
| 27.7 | 36.6 |
| 28.5 | 29.2 |
| 30.0 | 14.0 |
| 31.1 | 15.6 |
| 32.1 | 16.4 |
| 36.6 | 9.4 |
| 38.1 | 8.8 |
| 38.9 | 8.1 |

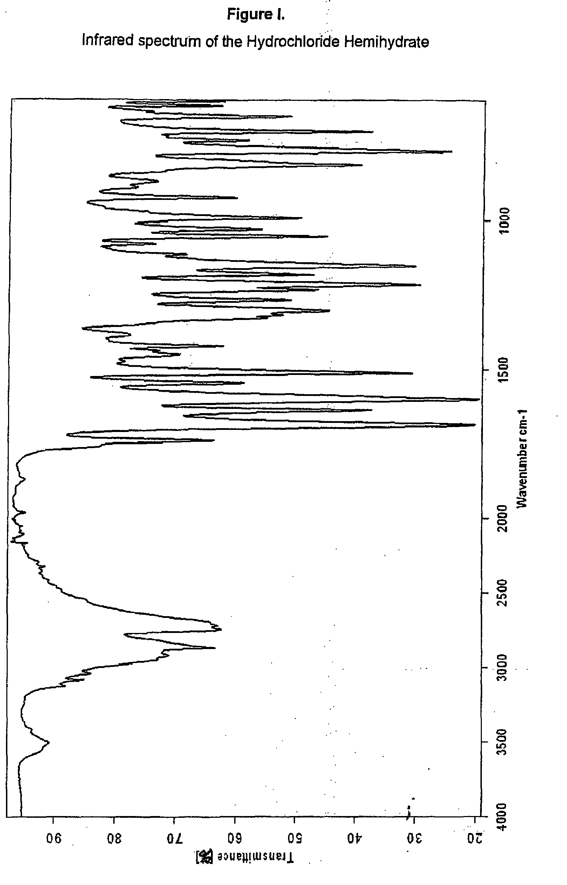

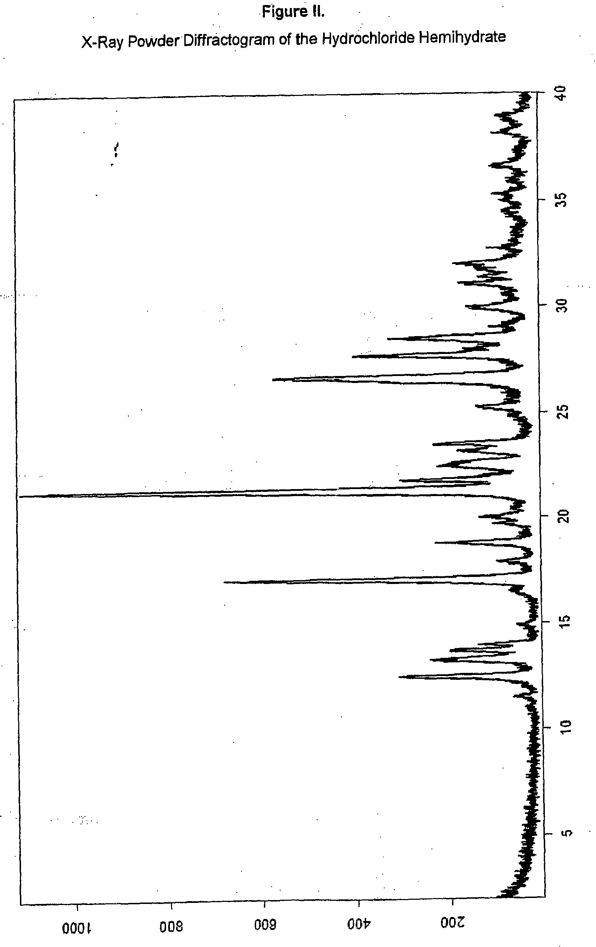

## Claims

1. 5-[4-[2-(N-methyl-N-(2-pyridyl)amino)ethoxy]benzyl]thiazolidine-2,4-dione, hydochloride hemihydrate : (rosiglitazone hydrochloride hemihydrate) **characterized in that** it:
(i) comprises water in the range of from about 1.0 to about 2.5% w/w, and
(ii) provides an infrared spectrum containing peaks at about 3504, 2751, 1688, 1601 and 1215 cm⁻¹, and/or
(iii) provides an X-ray powder diffraction (XRPD) pattern containing peaks at about 12.5, 17.1, 21.3, 26.7, 27.7 °2θ.

2. A compound according to claim 1, wherein the hydrochloride hemihydrate provides an infrared spectrum substantially in accordance with Figure I.

3. A compound according to claim 1, wherein the hydrochloride hemihydrate provides an X-ray powder diffraction (XRPD) pattern substantially in accordance with Table 2 and Figure II.

4. A compound according to claim 1; wherein the hydrochloride hemihydrate is in pure form.

5. A compound according to claim 1, wherein the hydrochloride hemihydrate is in a crystalline form.

6. A process for preparing rosiglitazone hydrochloride hemihydrate according to claim 1, which comprises reacting 5-[4-[2-(N-methyl-N-(2-pyridyl)amino)ethoxy]benzyl]thiazolidine-2,4-dione or an acid addition salt thereof in the presence of a suitable organic solvent and a suitable amount of water for formation of rosiglitazone hydrochloride hemihydrate, and thereafter the desired compound is recovered.

7. A compound according to any one of claims 1 to 5, for use in the treatment and/or prophylaxis of diabetes mellitus, conditions associated with diabetes mellitus and certain complications thereof.

8. A pharmaceutical composition comprising a therapeutially effective amount of rosiglitazone hydrochloride hemihydrate according to any cliams 1 to 5, and a pharmaceutically acceptable carrier.

9. A pharmaceutical composition comprising rosiglitazone hidrochloride hemihydrate according to any of the claims 1 to 5, in combination with one or more other known antidiabetic agents and a pharmaceutically acceptable carrier.

10. Use of rosiglitazone hydrochloride hemihydrate according to any of the claims 1 to 5 for the manufacture of a medicament for the treatment and/or prophylaxis of diabetes mellitus and certain complications thereof.

11. A method for the treatment and/prophylaxis of diabetes mellitus, conditions associated with diabetes mellitus and certain complications thereof in a human or non-human mammal, which comprises administering a therapeutically effective non-toxic amount of rosiglitazone hydrochloride hemihydrate to said mammal in a need thereof.
